(19) Europäisches Patentamt
European Patent Office
Office européen des brevets

(11) EP 1 653 934 B1

(12) EUROPEAN PATENT SPECIFICATION

(45) Date of publication and mention of the grant of the patent:
**14.05.2008 Bulletin 2008/20**

(21) Application number: **04769332.0**

(22) Date of filing: **16.08.2004**

(51) Int Cl.:
***A61K 31/00*** (2006.01) ***A61P 3/10*** (2006.01)

(86) International application number:
**PCT/IB2004/002934**

(87) International publication number:
**WO 2005/016323 (24.02.2005 Gazette 2005/08)**

# (54) USE OF C-KIT INHIBITORS FOR TREATING TYPE II DIABETES

VERWENDUNG VON C-KIT-INHIBITOREN FÜR DIE BEHANDLUNG VON TYP-2-DIABETES

UTILISATION D'INHIBITEURS DE C-KIT POUR LE TRAITEMENT DU DIABETE DE TYPE II

(84) Designated Contracting States:
**AT BE BG CH CY CZ DE DK EE ES FI FR GB GR HU IE IT LI LU MC NL PL PT RO SE SI SK TR**

(30) Priority: **15.08.2003 US 495088 P**

(43) Date of publication of application:
**10.05.2006 Bulletin 2006/19**

(73) Proprietor: **AB Science**
**75008 Paris (FR)**

(72) Inventors:
- **MOUSSY, Alain**
  **F-75006 Paris (FR)**
- **KINET, Jean-Pierre**
  **Lexington, MA 02421 (US)**

(74) Representative: **Warcoin, Jacques et al**
**Cabinet Régimbeau**
**20, rue de Chazelles**
**75847 Paris cedex 17 (FR)**

(56) References cited:

| | |
|---|---|
| EP-A- 0 934 931 | WO-A-01/64200 |
| WO-A-20/04014903 | WO-A-20/04076693 |
| WO-A-20/04098612 | WO-A-20/04105763 |

- **MA Y ET AL: "INDOLINONE DERIVATIVES INHIBIT CONSTITUTIVELY ACTIVATED KIT MUTANTS AND KILL NEOPLASTIC MAST CELLS" JOURNAL OF INVESTIGATIVE DERMATOLOGY, NEW YORK, NY, US, vol. 114, no. 2, February 2001 (2001-02), pages 392-394, XP001127437 ISSN: 0022-202X**
- **ZERMATI Y ET AL: "Effect of tyrosine kinase inhibitor STI571 on the kinase activity of wild-type and various mutated c - kit receptors found i mast cell neoplasms" ONCOGENE, BASINGSTOKE, HANTS, GB, vol. 22, 2003, pages 660-664, XP002286458 ISSN: 0950-9232**
- **BRECCIA ET AL: "Reduction of glycosilated haemoglobin with stable plasma insulin level in a ph+ cml diabetic patient responsive to imatinib" HAEMATOLOGICA/THE HEMATOLOGY JOURNAL (ONLINE), vol. 90, 2005,**
- **LASSILA ET AL: "Imatinib Attenuates Diabetes-Associated Atherosclerosis" ARTERIOSCLEROSIS THROMBOSIS AND VASCULAR BIOLOGY, vol. 24, no. 5, May 2004 (2004-05), pages 935-942,**
- **KADOWAKI TAKASHI; KUBOTA NAOTO ENZYMOLOGY; DIABETES MELLITUS, TYPE 2: COMPLICATIONS, ENZYMOLOGY;: "Protective role of imatinib in atherosclerosis." ARTERIOSCLEROSIS, THROMBOSIS, AND VASCULAR BIOLOGY, no. 24, 2005, pages 801-803,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 653 934 B1

## Description

The present invention relates to a method for treating type II diabetes, obesity and related disorders comprising administering a compound capable of depleting mast cells or a compound inhibiting mast cells degranulation, to a human in need of such treatment. Such compounds can be chosen from c-kit inhibitors and more particularly non-toxic, selective and potent c-kit inhibitors. Preferably, said inhibitor is unable to promote death of IL-3 dependent cells cultured in presence of IL-3.

Non-insulin-dependent diabetes mellitus (NIDDM), also known as type II diabetes, is defined as a chronic disease appearing when the insulin turns out to be inefficient in promoting glucose uptake by cells, which results in increased levels of glucose in the blood. This disease affects about 100 million people world-wide, 75% of which are obese at the time of diagnosis.

Diminution in the ability of the cells to respond adequately to insulin is often referred as insulin resistance. Excessive weight and lack of physical activity are regarded as being responsible for inducing insulin resistance. Over many years, the failure of the glucose uptake regulation leads to the development of Type II diabetes and the blood glucose level needs to be regulated with medicinal products. Ultimately, unregulated blood glucose level is responsible for blood vessels, kidney and eye damages, as well as cardiovascular diseases. This tissue damages contribute to mortality in diabetics.

Hypoglycemic agents such as sulfonylureas work by triggering the pancreas to make more insulin, which lower blood glucose. The side effects of sulfonylureas include hypoglycemia, renal and hepatic disease, gastrointestinal disturbances, increased cardiovascular mortality, dermatological reactions, drowsiness and headache. Biguanides lower blood glucose levels by reducing intestinal glucose absorption and hepatic glucose, but not by stimulating insulin secretion. The major side effects of biguanidine are lactic acidosis and increased cardiovascular mortality. Alpha-glucosidase inhibitors decrease the absorption of carbohydrates from the digestive tract, thereby lowering the after-meal glucose level, but gastrointestinal side effects and hypoglycemia are observed. Thiazolidinediones, such as rosiglitazone are PPARgamma agonists and increase the cell's sensitivity to insulin. However, they may be responsible for water retention, liver diseases, cardiovascular diseases, red blood cell abnormalities, and headache.

WO 01/64200 describes the use of CGP57148B for the treatment of diabetic retinopathy which is a c-kit inhibitor (Zermati et al, 2003, Oncogene, Basingstoke, Hants, GB, Vol 22 pages 660-664). Besides, WO 2004/105763 and its priority GB 0312086.2 of May, 27 2003 disclose the use of CGP57148B for treating type I diabetes. At last, WO 2004/014903, WO 2004/098612 and WO 2004/076693 relates to aryl-aminothiazole compounds for treating type II diabetes and obesity.

Because treatment of Type II diabetes requires long term administration of compounds lowering blood glucose level, there is still a great need for improved and safer methods.

In connection with the present invention, we have unexpectedly discovered that c-kit inhibitors lower the level of glucose, cholesterol, triglycerides and non esterified fatty acids in blood.

In addition, these inhibitors do not affect significantly the level of insulin contrary to compounds of the thiazolidinedione family.

This observation is surprising and we can only speculate at this time of the mechanism of action of c-kit inhibitors. We know that c-kit is of crucial importance for activation of mast cells. Following mast cells activation, released granules liberate various factors which could directly or indirectly participate in the regulation of different metabolites uptake and processing by the cells. Among such factors, we can cite a cocktail of different proteases, lipid-derived mediators (prostaglandins, thromboxanes and leucotrienes) and various cytokines (IL-1, IL-2, IL-3, IL-4, IL-5, IL-6, IL-8, TNF-$\alpha$, GM-CSF, MIP-1a, MIP-1b, MIP-2 and IFN-$\gamma$). In Lyon CJ, et al, Proc Nutr Soc 2001 Aug;60(3):329-39 is it mentioned that adipose tissue is a dynamic endocrine organ that secretes a number of factors that are increasingly recognized to contribute to systemic and vascular inflammation.

The major secretory compartment of adipose tissue consists of adipocytes, fibroblasts, and mast cells. These cells, using endocrine, paracrine and autocrine pathways, secrete multiple bioactive molecules, conceptualized as "adipokines".

Here, based on our observation that c-kit inhibitors works in lowering notably blood glucose, we postulate that mast cells regulate, directly or indirectly, a number of the processes that contribute to the development of atherosclerosis, including hypercholesterolemia, hypergycemia, hypertension, endothelial dysfunction, insulin resistance, and vascular remodeling. But, as this point, other mechanisms may not be ruled out.

A new route for treating type II diabetes, obesity and related disorders is provided, which consists of administering c-kit inhibitors to patients.

## Description

The present invention relates to the use of N-phenyl-2-pyrimidine-amine derivatives selected from the compounds corresponding to formula II :

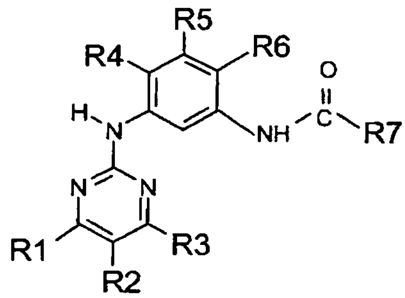

**Formula II**

Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function, for manufacturing a medicament for preventing, delaying the onset and/or treating type II diabetes, obesity, hypercholesterolemia, hypergycemia, hypertension, endothelial dysfunction, insulin resistance, and vascular remodelling in human.

Preferably, R7 is the following group :

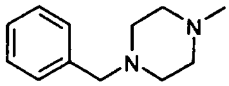

Among these compounds, the preferred are defined as follows :

R1 is a heterocyclic group, especially a pyridyl group,
R2 and R3 are H,
R4 is a C1-C3 alkyl, especially a methyl group,
R5 and R6 are H,
and R7 is a phenyl group bearing at least one substituent, which in turn possesses at least one

basic site, such as an amino function, for example the group :

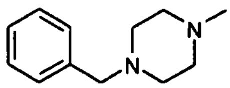

In a preferred embodiment, the invention relates to a method for treating type II diabetes comprising the administration of an effective amount of the compound known in the art as CGP57148B :

4-(4-méhylpipérazine-1-ylméthyl)-N-[4-méthyl-3-(4-pyridine-3-yl)pyrimidine-2 ylamino)phényl]-benzamide corresponding to the following formula :

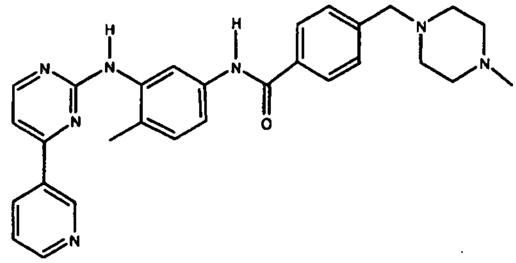

The preparation of this compound is described in example 21 of EP 564 409 and the β-form, which is particularly useful is described in WO 99/03854.

The use according to the invention includes preventing, delaying the onset and/or treating type II diabetes and associated damages in humans.

Therefore, the invention embraces the use of the compounds defined above to manufacture a medicament for treating type II diabetes including obesity, hypercholesterolemia, hypergycemia, hypertension, endothelial dysfunction, insulin resistance, and vascular remodelling.

More particularly, the above compounds are useful for preventing the onset or development of type II diabetes in obese persons.

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

More particularly, the invention relates to a pharmaceutical composition intended for oral administration.

Pharmaceutical compositions suitable for use in the invention include compositions wherein compounds for depleting mast cells, such as c-kit inhibitors, or compounds inhibiting mast cells degranulation are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred.

## Example 1: Effect of different c-kit inhibitors on serum glucose, insulin, triglycerides, cholesterol and non esterified fatty acids levels in db/db mice

### 1.1 PURPOSE OF THE STUDY

The objective of this study is to assess the effects of different c-kit inhibitors on serum glucose, insulin, triglycerides, cholesterol and non esterified fatty acids levels in male db/db mice dosed orally, once-a-day, for 5 days.

### 1.2 MATERIALS AND METHODS

1.2.2 Test system

30 male 9/11 weeks old C57BL/Ks J Rj-db (db/db) mice (Janvier, France or Harlan, France), weighing in the target range of 30 to 50 g, will be included in this study.
They will be housed in a temperature (19.5-24.5°C) and relative humidity (45-65%) controlled room with a 12-h light/dark cycle, with ad libitum access to filtered tap-water and irradiated pelleted laboratory chow (ref. A04, U.A.R., France) throughout the study. Upon receipt at animal facilities, they will be housed 5 per cage and at least a 5-day acclimatization period will be observed. Animals will be individually identified on the tail.

1.2.3 Study materials

- Substance tested code: substances N° 1 and N°2
- Reference substance
  Code : rosiglitazone 10 mg/kg
  Source : Sequoia Research Products Ltd, UK
- Vehicle
  The vehicle will be defined by the Sponsor but, if no indication is supplied, a 3% Arabic gum aqueous solution (w/v) will be used.
- Reagents
  - Glucose kit (ref. 442640, Beckman Coulter, France)
  - Insulin ELIT Plus kit (ref. INSRAT01-8N, Eurobio, France)
  - Triglycerides kit (ref. 445850, Beckman Coulter, France)
  - Total cholesterol kit (ref. 467825, Beckman Coulter, France)
  - Non esterified fatty acids kit (NEFA, ref. FA115, Randox, France)
  - Isoflurane (Forene®, Abbott, UK)
- Principal Equipment
  - Balances (AT261 model, Mettler, France)
  - Centrifuge (2K15 model, Sigma, France)
  - Multi-parametric analyzer (Synchron CX-4 model, Beckman Coulter, France)
  - Microplate reader (Multiskan RC model, Labsystem, France)
- Principal Data Processing Systems
  Excel (Microsoft v.2000), Biolise (Labsystem v.2.65) and SigmaStat (SPSS v.2.03)

1.2.4 Study design

6 groups of 5 animals each will be included in this study :

Group 1 : vehicle
Group 2 : test substance N°1, dose 1, route of administration
Group 3 : test substance N°1, dose 2, route of administration
Group 4 : test substance N°2, dose 1, route of administration
Group 5 : test substance N°2, dose 2, route of administration
Group 6 : rosiglitazone, 10 mg/kg, route of administration
The doses will be expressed in terms of free active substance.

The test and reference substances will be extemporaneously prepared as instructed.
The test and reference substances and the vehicle will be administered in a volume of 5 ml/kg adjusted according to individual body weight values.

1.2.5 Experimental protocol

One to three days before beginning the treatments (TO), mice will be weighed and blood samples will be collected through the retro-orbital plexus under isoflurane anesthesia. Blood samples will be kept at room temperature for 5 to 10 min to form a spontaneous clot, then put in ice until they are centrifuged at 3500 x g for 10-15 min at 4 °C. An aliquot of serum will be used for measuring glucose levels.
Six groups of 5 mice will be formed with respect to homogeneous glycemia values by using a randomization

table. Animal showing glycemia below 20 mM will be excluded from the study.

From T1 to T5, the mice will be weighed and dosed once daily for 5 consecutive days at constant time.

At T5, 2 hours after the last administration, blood samples will be collected through the retro-orbital plexus under isoflurane anesthesia. Blood samples will be kept at room temperature for 5 to 10 min to form a spontaneous clot, then put in ice until they are centrifuged at 3500 x g for 10 min at 4 °C. Serum will be aliquoted and frozen at -20°C until use. After blood collection, the mice will be euthanized by cervical dislocation.

Serum glucose and triglycerides levels will be determined using the Synchron CX4 analyzer. Serum non esterified fatty acids levels will be measured manually using a colorimetric method, and insulin levels determined by ELISA.

1.2.6 Analysis and expression of results

The results will be expressed as mean ± SEM :

- Glucose levels (mM) at T5
- Insulin levels (nM) at T5
- Triglycerides levels (mM) at T5
- Non esterified fatty acids levels (mM) at T5

For each parameter, a % of effect will be calculated according to following formula:

$$((\text{vehicle group} - \text{test group}) / \text{vehicle group}) * 100$$

Statistical analysis will consist in a one-way analysis of variance followed by multiple comparisons versus the vehicle group (Dunnett's test). In case the equal variance test fails, a Kruskall-Wallis one-way analysis of variance on ranks will be proposed. A difference will be considered significant for $p<0.05$.

This study has been performed according to the model described in Grasa et al. , Oleoyl-estrone lowers the body weight of ob/ob and db/db mice. Horm. Metab. Res. 2000, 32, 246-250 and has be subjected to Quality Assurance monitoring.

**3. RESULTS : Effects of AB compounds on serum biomarkers in male db/db mice dosed P.O. for 5 days**

**Table 1 :**

| **Treatment (mg/kg)** | **Vehicle** (n=5) | **N°1 100** (n=5) | **N°1 200** (n=5) |
|---|---|---|---|
| **Glucose (mM)** | 27,46 ± 2,95 | 21,90 ± 3,01 | 19,72 ± 1,23 |
| Effect (%) | | 20 | 28 |

| | | | |
|---|---|---|---|
| **Cholesterol (mM)** | 3,38 ± 0,08 | 3,37 ± 0,12 | 2,79 ± 0,29 |
| Effect (%) | | 0 | 17 |
| **Triglycerides (mM)** | 1,57 ± 0,11 | 1,00 ± 0.08 ** | 0,89 ± 0.06 ** |
| Effect (%) | | 36 | 43 |
| **NEFA (mM)** | 0,82 ± 0,05 | 0,68 ± 0,14 | 0,79 ± 0,084 |
| Effect (%) | | 17 | 3 |
| **Insulin (nM)** | 3,69 ± 0,86 | 3,31 ± 0,89 | 2,06 ± 0,38 |
| Effect (%) | | 10 | 44 |

| **Treatment (mg/kg)** | **N°2 100** (n=5) | **N°2 200** (n=5) | **Rosiglitazone 10** (n=5) | **ANOVA (P value)** |
|---|---|---|---|---|
| **Glucose (mM)** | 18,16 ± 2,22 | 15,64 ± 3.23 * | 11,25 ± 2.65 ** | **0,006** |
| Effect (%) | 34 | 43 | 59 | |
| **Cholesterol (mM)** | 3,24 ± 0,09 | 2,72 ± 0,13 | 2,74 ± 0,47 | **0,163** |
| Effect (%) | 4 | 19 | 19 | |
| **Triglycerides (mM)** | 0,77 ± 0.08 ** | 0,66 ± 0.05 ** | 0,46 ± 0.10 ** | **< 0.001** |
| Effect (%) | 51 | 58 | 71 | |
| **NEFA (mM)** | 0,70 ± 0,048 | 0,71 ± 0,07 | 0,31 ± 0.061 * | **0.036 (°)** |
| Effect (%) | 14 | 13 | 63 | |

| | | | | |
|---|---|---|---|---|
| **Insulin (nM)** | 2,15 ± 0,42 | 2,82 ± 0,59 | 0,82 ± 0.27 * | **0,039** |
| Effect (%) | 42 | 24 | 77 | |

Values are expressed as mean ± SEM

Statistics : One-way analysis of variance (ANOVA) followed by a Dunnett's test.

or (°) Kruskal Wallis analysis of variance on the ranks followed by a Dunn's test.

*$p<0.05$, ** $p<0.01$ as compared to vehicle

Effect % as compared to vehicle

**Claims**

1. The use of a compound selected from the group consisting of N-phenyl-2-pyrimidine-amine derivatives having the formula II:

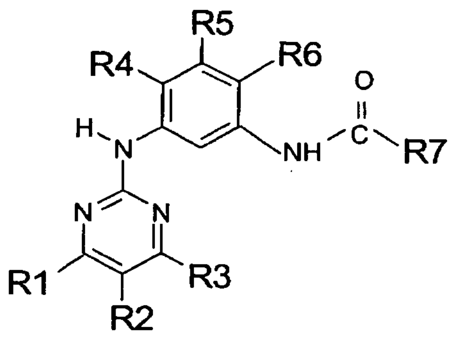

Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function,
for manufacturing a medicament for preventing, delaying the onset and/or treating type II diabetes, obesity, hypercholesterolemia, hypergycemia, hypertension, endothelial dysfunction, insulin resistance, and vascular remodelling in human.

2. The use according to claim 1 wherein R7 is the following group :

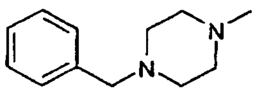

3. The use of 4-(4-mehylpiperazin-1-ylmethyl)-N-[4-methyl-3-(4-pyridine-3-yl)pyrimidine-2 ylamino)phenyl]-benzamide of formula :

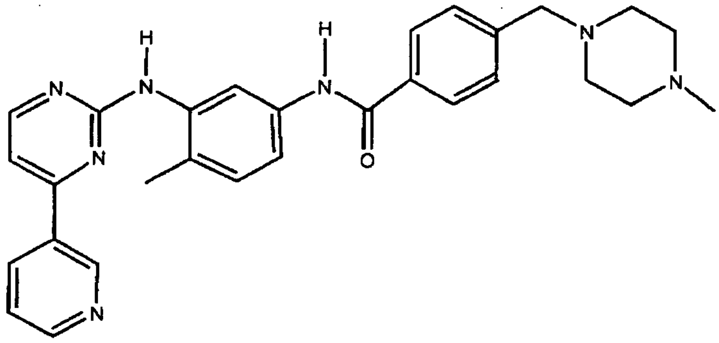

for manufacturing a medicament for preventing, delaying the onset and/or treating type II diabetes in human.

4. The use of 4-(4-mehylpiperazin-1-ylmethyl)-N-j4-methyl-3-(4-pyridine-3-yl)pyrimidine-2 ylamino)phenyl]-benzamide for manufacturing a medicament for preventing, delaying the onset and/or treating obesity in human.

5. The use of 4-(4-mehylpiperazin-1-ylmethyl)-N-[4-methyl-3-(4-pyridine-3-yl)pyrimidine-2 ylamino)phenyl]-benzamide for manufacturing a medicament for preventing, delaying the onset and/or treating hypercholesterolemia in human.

6. The use of 4-(4-mehylpiperazin-1-ylmethyl)-N-[4-methyl-3-(4-pyridine-3-yl)pyrimidine-2 ylamino)phenyl]-benzamide for manufacturing a medicament for preventing, delaying the onset and/or treating hypertension in human.

7. The use of 4-(4-mehylpiperazin-1-ylinethyl)-N-[4-methyl-3-(4-pyridine-3-yl)pyrimidine-2 ylamino)phenyl]-benzamide for manufacturing a medicament for preventing, delaying the onset and/or treating endothelial dysfunction in human.

**Patentansprüche**

1. Verwendung einer Verbindung, ausgewählt aus der Gruppe bestehend aus N-Phenyl-2-pyrimidinamin-Derivaten mit der Formel II:

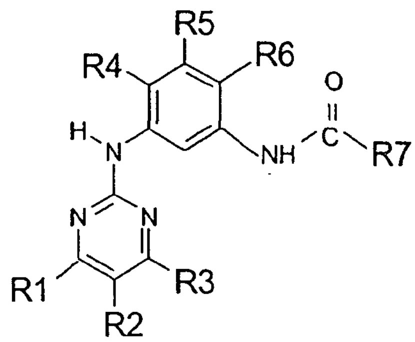

wobei, R1, R2 und R3 unabhängig ausgewählt sind aus H, F, Cl, Br, I und einem Cl-C5-Alkyl oder einer cyclischen oder heterocyclischen Gruppe, insbesondere einer Pyridylgruppe;
R4, R5 und R6 unabhängig ausgewählt sind aus H, F, Cl, Br, I und einem C1-C5-Alkyl, insbesondere einer Methylgruppe;
und R7 eine Phenylgruppe ist, die wenigstens einen Substituenten aufweist, der wiederum wenigstens eine basische Stelle besitzt, wie beispielsweise eine Aminofunktion,
zur Herstellung eines Arzneimittels zum Verhindern, Verzögern des Beginns und/oder Behandeln von Diabetes Typ II, Fettleibigkeit, Hypercholesterinämie, Hyperglykämie, Bluthochdruck, Endotheldysfunktion, Insulinresistenz und Gefäßnachbildung beim Menschen.

2. Verwendung nach Anspruch 1, wobei R7 die folgende Gruppe ist:

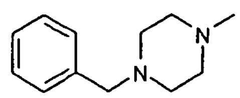

3. Verwendung von 4-(4-Methylpiperazin-1-ylmethyl)-N-[4-methyl-3-(4-pyridin-3-yl)pyrimidin-2 ylamino)phenyl]-benzamid der Formel:

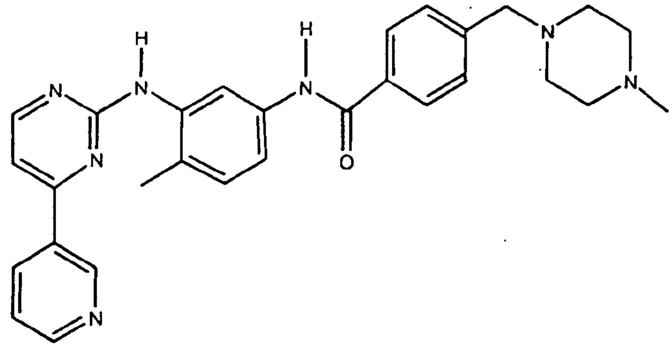

zur Herstellung eines Arzneimittels zum Verhindern, Verzögern des Beginns und/oder Behandeln von Diabetes Typ II beim Menschen.

**4.** Verwendung von 4-(9-Methylpiperazin-1-ylmethyl)-N-[4-methyl-3-(4-pyridin-3-yl)pyrimidin-2 ylamino)phenyl]-benzamid zur Herstellung eines Arzneimittels zum Verhindern, Verzögern des Beginns und/oder Behandeln von Fettleibigkeit beim Menschen.

**5.** Verwendung von 4-(4-Methylpiperazin-1-ylmethyl)-N-[4-methyl-3-(4-pyridin-3-yl)pyrimidin-2 ylamino)phenyl]-benzamid zur Herstellung eines Arzneimittels zum Verhindern, Verzögern des Beginns und/oder Behandeln von Hypercholesterinämie beim Menschen.

**6.** Verwendung von 4-(4-Methylpiperazin-1-ylmethyl)-N-[4-methyl-3-(4-pyridin-3-yl)pyrimidin-2 ylamino)phenyl]-benzamid zur Herstellung eines Arzneimittels zum Verhindern, Verzögern des Beginns und/oder Behandeln von Bluthochdruck beim Menschen.

**7.** Verwendung von 4-(4-Methylpiperazin-1-ylmethyl)-N-[4-methyl-3-(4-pyridin-3-yl)pyrimidin-2 ylamino)phenyl]-benzamid zur Herstellung eines Arzneimittels zum Verhindern, Verzögern des Beginns und/oder Behandeln von Endotheldysfunktion beim Menschen.

## Revendications

**1.** Utilisation d'un composé choisi dans l'ensemble constitué par les dérivés de N-phényl-2-pyrimidine-amine de formule (II) :

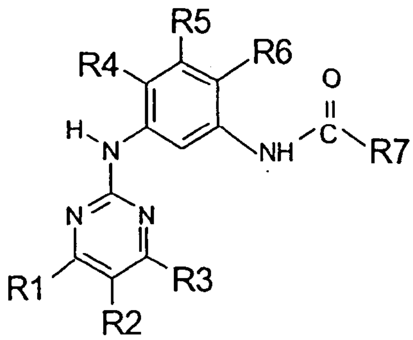

dans laquelle R1, R2 et R3 sont indépendamment choisis parmi H, F, Cl, Br, I, un groupe alkyle en C1-C5 ou un groupe cyclique ou hétérocyclique, en particulier un groupe pyridyle ;
R4, R5 et R6 sont indépendamment choisis parmi H, F, C1, Br, I, un groupe alkyle en C1-C5, en particulier un groupe méthyle ;
et R7 est un groupe phényle portant au moins un substituant, qui à son tour possède au moins un site basique, tel

qu'une fonctionnalité amino,
pour la fabrication d'un médicament destiné à prévenir, retarder l'apparition et/ou traiter le diabète de type II, l'obésité, l'hypercholestérolémie, l'hyperglycémie, l'hypertension, un dysfonctionnement endothélial, une résistance à l'insuline, et un remodelage vasculaire chez l'être humain.

2. Utilisation selon la revendication 1, dans laquelle R7 est le groupe suivant :

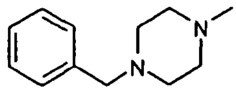

3. Utilisation de 4-(4-méthylpipérazin-1-yl-méthyl)-N-[4-méthyl-3-(4-pyridin-3-yl)pyrimidin-2-yl-amino)phényl]benzamide de formule :

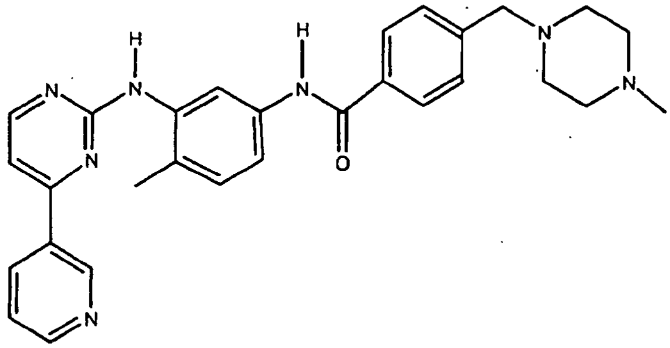

pour la fabrication d'un médicament destiné à prévenir, retarder l'apparition et/ou traiter le diabète de type II chez l'être humain.

4. Utilisation de 4-(4-méthylpipérazin-1-yl-méthyl)-N-[4-méthyl-3-(4-pyridin-3-yl)pyrimidin-2-yl-amino)phényl]benzamide pour la fabrication d'un médicament destiné à prévenir, retarder l'apparition et/ou traiter l'obésité chez l'être humain.

5. Utilisation de 4-(4-méthylpipérazin-1-yl-méthyl)-N-[4-méthyl-3-(4-pyridin-3-yl)pyrimidin-2-yl-amino)phényl]benzamide pour la fabrication d'un médicament destiné à prévenir, retarder l'apparition et/ou traiter l'hypercholestérolémie chez l'être humain.

6. Utilisation de 4-(4-méthylpipérazin-1-yl-méthyl)-N-[4-méthyl-3-(4-pyridin-3-yl)pyrimidin-2-yl-amino)phényl]benzamide pour la fabrication d'un médicament destiné à prévenir, retarder l'apparition et/ou traiter l'hypertension chez l'être humain.

7. Utilisation de 4-(4-méthylpipérazin-1-yl-méthyl)-N-[4-méthyl-3-(4-pyridin-3-yl)pyrimidin-2-yl-amino)phényl]benzamide pour la fabrication d'un médicament destiné à prévenir, retarder l'apparition et/ou traiter un dysfonctionnement endothélial chez l'être humain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0164200 A
- WO 2004105763 A
- GB 0312086 A
- WO 2004014903 A
- WO 2004098612 A
- WO 2004076693 A
- EP 564409 A
- WO 9903854 A
- US 60495088 B


### Non-patent literature cited in the description


- **ZERMATI et al.** *Oncogene,* 2003, vol. 22, 660-664
- **LYON CJ et al.** *Proc Nutr Soc,* August 2001, vol. 60 (3), 329-39
- Remington's Pharmaceutical Sciences. Maack Publishing Co,
- *Horm. Metab. Res.,* 2000, vol. 32, 246-250

SEQUENCE LISTING

<110> AB Science

<120> Use of c-kit inhibitors for treating type II diabetes

<130> 346628 D21538 NT

<150> US 60/495,088
<151> 2003-08-15

<160> 5

<170> PatentIn Ver. 2.1

<210> 1
<211> 976
<212> PRT
<213> Homo sapiens

<220>
<223> Human c-kit

<400> 1

```
Met Arg Gly Ala Arg Gly Ala Trp Asp Phe Leu Cys Val Leu Leu Leu
1               5                   10                  15

Leu Leu Arg Val Gln Thr Gly Ser Ser Gln Pro Ser Val Ser Pro Gly
            20                  25                  30

Glu Pro Ser Pro Pro Ser Ile His Pro Gly Lys Ser Asp Leu Ile Val
        35                  40                  45

Arg Val Gly Asp Glu Ile Arg Leu Leu Cys Thr Asp Pro Gly Phe Val
    50                  55                  60

Lys Trp Thr Phe Glu Ile Leu Asp Glu Thr Asn Glu Asn Lys Gln Asn
65                  70                  75                  80

Glu Trp Ile Thr Glu Lys Ala Glu Ala Thr Asn Thr Gly Lys Tyr Thr
                85                  90                  95

Cys Thr Asn Lys His Gly Leu Ser Asn Ser Ile Tyr Val Phe Val Arg
            100                 105                 110

Asp Pro Ala Lys Leu Phe Leu Val Asp Arg Ser Leu Tyr Gly Lys Glu
        115                 120                 125

Asp Asn Asp Thr Leu Val Arg Cys Pro Leu Thr Asp Pro Glu Val Thr
    130                 135                 140

Asn Tyr Ser Leu Lys Gly Cys Gln Gly Lys Pro Leu Pro Lys Asp Leu
145                 150                 155                 160

Arg Phe Ile Pro Asp Pro Lys Ala Gly Ile Met Ile Lys Ser Val Lys
                165                 170                 175

Arg Ala Tyr His Arg Leu Cys Leu His Cys Ser Val Asp Gln Glu Gly
            180                 185                 190

Lys Ser Val Leu Ser Glu Lys Phe Ile Leu Lys Val Arg Pro Ala Phe
        195                 200                 205

Lys Ala Val Pro Val Val Ser Val Ser Lys Ala Ser Tyr Leu Leu Arg
    210                 215                 220
```

```
Glu Gly Glu Glu Phe Thr Val Thr Cys Thr Ile Lys Asp Val Ser Ser
225                 230                 235                 240

Ser Val Tyr Ser Thr Trp Lys Arg Glu Asn Ser Gln Thr Lys Leu Gln
                245                 250                 255

Glu Lys Tyr Asn Ser Trp His His Gly Asp Phe Asn Tyr Glu Arg Gln
            260                 265                 270

Ala Thr Leu Thr Ile Ser Ser Ala Arg Val Asn Asp Ser Gly Val Phe
        275                 280                 285

Met Cys Tyr Ala Asn Asn Thr Phe Gly Ser Ala Asn Val Thr Thr Thr
    290                 295                 300

Leu Glu Val Val Asp Lys Gly Phe Ile Asn Ile Phe Pro Met Ile Asn
305                 310                 315                 320

Thr Thr Val Phe Val Asn Asp Gly Glu Asn Val Asp Leu Ile Val Glu
                325                 330                 335

Tyr Glu Ala Phe Pro Lys Pro Glu His Gln Gln Trp Ile Tyr Met Asn
            340                 345                 350

Arg Thr Phe Thr Asp Lys Trp Glu Asp Tyr Pro Lys Ser Glu Asn Glu
        355                 360                 365

Ser Asn Ile Arg Tyr Val Ser Glu Leu His Leu Thr Arg Leu Lys Gly
    370                 375                 380

Thr Glu Gly Gly Thr Tyr Thr Phe Leu Val Ser Asn Ser Asp Val Asn
385                 390                 395                 400

Ala Ala Ile Ala Phe Asn Val Tyr Val Asn Thr Lys Pro Glu Ile Leu
                405                 410                 415

Thr Tyr Asp Arg Leu Val Asn Gly Met Leu Gln Cys Val Ala Ala Gly
            420                 425                 430

Phe Pro Glu Pro Thr Ile Asp Trp Tyr Phe Cys Pro Gly Thr Glu Gln
        435                 440                 445

Arg Cys Ser Ala Ser Val Leu Pro Val Asp Val Gln Thr Leu Asn Ser
    450                 455                 460

Ser Gly Pro Pro Phe Gly Lys Leu Val Val Gln Ser Ser Ile Asp Ser
465                 470                 475                 480

Ser Ala Phe Lys His Asn Gly Thr Val Glu Cys Lys Ala Tyr Asn Asp
                485                 490                 495

Val Gly Lys Thr Ser Ala Tyr Phe Asn Phe Ala Phe Lys Gly Asn Asn
            500                 505                 510

Lys Glu Gln Ile His Pro His Thr Leu Phe Thr Pro Leu Leu Ile Gly
        515                 520                 525

Phe Val Ile Val Ala Gly Met Met Cys Ile Ile Val Met Ile Leu Thr
    530                 535                 540

Tyr Lys Tyr Leu Gln Lys Pro Met Tyr Glu Val Gln Trp Lys Val Val
545                 550                 555                 560

Glu Glu Ile Asn Gly Asn Asn Tyr Val Tyr Ile Asp Pro Thr Gln Leu
                565                 570                 575
```

Pro Tyr Asp His Lys Trp Glu Phe Pro Arg Asn Arg Leu Ser Phe Gly
            580                 585                 590

Lys Thr Leu Gly Ala Gly Ala Phe Gly Lys Val Val Glu Ala Thr Ala
        595                 600                 605

Tyr Gly Leu Ile Lys Ser Asp Ala Ala Met Thr Val Ala Val Lys Met
    610                 615                 620

Leu Lys Pro Ser Ala His Leu Thr Glu Arg Glu Ala Leu Met Ser Glu
625                 630                 635                 640

Leu Lys Val Leu Ser Tyr Leu Gly Asn His Met Asn Ile Val Asn Leu
                645                 650                 655

Leu Gly Ala Cys Thr Ile Gly Gly Pro Thr Leu Val Ile Thr Glu Tyr
            660                 665                 670

Cys Cys Tyr Gly Asp Leu Leu Asn Phe Leu Arg Arg Lys Arg Asp Ser
        675                 680                 685

Phe Ile Cys Ser Lys Gln Glu Asp His Ala Glu Ala Ala Leu Tyr Lys
    690                 695                 700

Asn Leu Leu His Ser Lys Glu Ser Ser Cys Ser Asp Ser Thr Asn Glu
705                 710                 715                 720

Tyr Met Asp Met Lys Pro Gly Val Ser Tyr Val Val Pro Thr Lys Ala
                725                 730                 735

Asp Lys Arg Arg Ser Val Arg Ile Gly Ser Tyr Ile Glu Arg Asp Val
            740                 745                 750

Thr Pro Ala Ile Met Glu Asp Asp Glu Leu Ala Leu Asp Leu Glu Asp
        755                 760                 765

Leu Leu Ser Phe Ser Tyr Gln Val Ala Lys Gly Met Ala Phe Leu Ala
    770                 775                 780

Ser Lys Asn Cys Ile His Arg Asp Leu Ala Ala Arg Asn Ile Leu Leu
785                 790                 795                 800

Thr His Gly Arg Ile Thr Lys Ile Cys Asp Phe Gly Leu Ala Arg Asp
                805                 810                 815

Ile Lys Asn Asp Ser Asn Tyr Val Val Lys Gly Asn Ala Arg Leu Pro
            820                 825                 830

Val Lys Trp Met Ala Pro Glu Ser Ile Phe Asn Cys Val Tyr Thr Phe
        835                 840                 845

Glu Ser Asp Val Trp Ser Tyr Gly Ile Phe Leu Trp Glu Leu Phe Ser
    850                 855                 860

Leu Gly Ser Ser Pro Tyr Pro Gly Met Pro Val Asp Ser Lys Phe Tyr
865                 870                 875                 880

Lys Met Ile Lys Glu Gly Phe Arg Met Leu Ser Pro Glu His Ala Pro
                885                 890                 895

Ala Glu Met Tyr Asp Ile Met Lys Thr Cys Trp Asp Ala Asp Pro Leu
            900                 905                 910

Lys Arg Pro Thr Phe Lys Gln Ile Val Gln Leu Ile Glu Lys Gln Ile
        915                 920                 925

```
Ser Glu Ser Thr Asn His Ile Tyr Ser Asn Leu Ala Asn Cys Ser Pro
    930                 935                 940

Asn Arg Gln Lys Pro Val Val Asp His Ser Val Arg Ile Asn Ser Val
945                 950                 955                 960

Gly Ser Thr Ala Ser Ser Ser Gln Pro Leu Leu Val His Asp Asp Val
                965                 970                 975
```

<210> 2
<211> 30
<212> DNA
<213> Homo sapiens

<220>
<223> Primer

<400> 2
aagaagagat ggtacctcga ggggtgaccc 30

<210> 3
<211> 33
<212> DNA
<213> Homo sapiens

<220>
<223> Primer

<400> 3
ctgcttcgcg gccgcgttaa ctcttctcaa cca 33

<210> 4
<211> 20
<212> DNA
<213> Homo sapiens

<220>
<223> Primer

<400> 4
agctcgttta gtgaaccgtc 20

<210> 5
<211> 20
<212> DNA
<213> Homo sapiens

<220>
<223> Primer
<400> 5
gtcagacaaa atgatgcaac 20